# EUROPEAN PATENT APPLICATION

(11) **EP 3 575 042 A1**
(43) Date of publication of application: **04.12.2019**
(21) Application number: 18174881.5
(22) Date of filing: 29.05.2018
(51) Int. Cl.: B25J 9/00, A61F 2/64, A61F 2/68, B25J 9/10, A61F 2/50, A61H 3/00, A61H 1/02

(54) **DEVICE COMPRISING INPUT AND OUTPUT PULLEYS ON NONPARALLEL OR PARALLEL AND MUTUALLY HINGEABLE AXES**

(71) Applicant: Vrije Universiteit Brussel, 1050 Brussel (BE)
(72) Inventor: Lefeber, Dirk, 2870 Puurs (BE); Rodríguez Cianca, David, 28804 Alcalá de Henares (Madrid) (ES)
(74) Representative: Brantsandpatents bvba

(57) **Abstract**

The current invention concerns a device and a method for transmitting torque. The device may comprise an elongated and bendable torque transmission member, e.g. a cable, and a hinge element along which the member is guided via one or more guide pulleys. The hinge element may comprise a first and a second base element hingedly attached and comprising a first hinge axis. The first element may comprise a first guide pulley with pulley rotational axis in essence perpendicular to the first hinge axis. The second element may comprise a second guide pulley with pulley rotational axis in essence perpendicular to the first hinge axis. The positions of the first guide pulley in the first element and the second guide pulley in the second element may be configured to guide a portion of the member in essence coaxial to the hinge axis. In a preferred embodiment, the device is a wearable robot, e.g. an exoskeleton.

## Description

### Technical field

The invention may pertain to power input and output pulleys on nonparallel axes (US patent subclass 474/61). The invention may pertain to the technical field of wearable robots, e.g. exoskeletons, and in particular to prostheses not implantable in the body (IPC A61F 2/50).

### Background

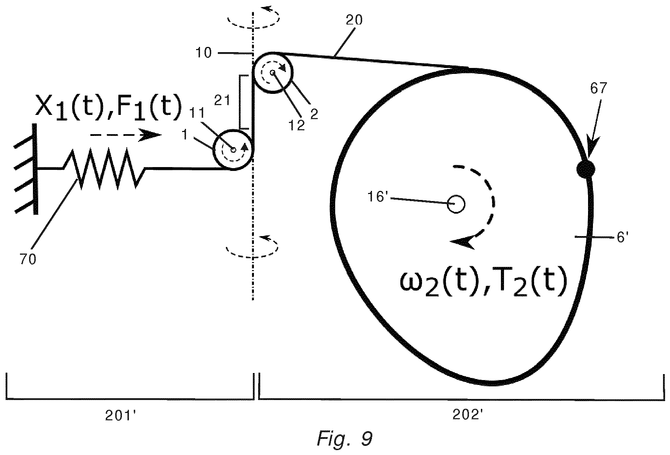

US 2012/0 179 075 A1 discloses an exoskeleton comprising at least one pulley-cable pair for torque application to a distal exoskeleton joint. Figure 9A of the document discloses cable routing via a proximal joint idler pulley, which is coaxial to the rotation axis of the proximal joint.

F. Moon, "Model: V07 Belt and Pulley Drive Between Two Non-Parallel axes", http://kmoddl.library.cornell.edu/model.php?m=295, 1 July 2003, discloses a model which demonstrates how to transmit motion from one axis of rotation to another non-parallel axis of rotation in the same plane. The mechanism uses intermediate guide pulley wheels on a vertical axis perpendicular to the plane of the other two axes. The guide pulley wheels thereby comprise an axis of rotation parallel to said vertical axis.

A wearable robot may comprise a joint with a joint rotational axis. A torque transmission system based on cables and pulleys to transfer power over said joint, whereby a guide pulley is provided with pulley rotational axis parallel to the joint rotational axis, has a dimension in all directions perpendicular to the joint rotational axis of at least the pulley diameter. Wearable robots which large spatial extents are undesired, as they may spatially and/or inertially hinder a user. Some embodiments of such a joint may additionally provide a tension on a cable which depends on the angular configuration of the joint, as the path length of the cable is changed with the angular configuration of the joint, a further undesired effect.

The present invention aims to resolve at least some of the problems mentioned above.

### Summary of the invention

The present invention provides a wearable robot, according to claim 1.

The present invention provides a device, according to claim 13.

The present invention provides a method for transmitting torque, according to claim 14.

The present invention provides a solution to the technical problem of torque transmission over mutually hingable and nonparallel input and output axes. The present invention is advantageous as it allows for limited spatial extent of a hinge element in a direction perpendicular to the first hinge axis. The present invention is additionally advantageous as the tension on the member, e.g. cable, is in essence independent, up to torsion of the member, of the angular configuration of the hinge element, because the length of the path of the member is in essence independent of the angular configuration of the hinge element.

### Description of figures

**Figures 1****,** **2****,** **3****,** **6** **and** **7** show schematic representations of embodiments of hinge elements of devices according to the present invention.
**Figure 4** shows a perspective view of an embodiment of a hinge element according to the present invention.
**Figure 5** shows a schematic representation of a wearable robot according to the present invention.
**Figures 8** **and** **9** show schematic representations of alternative embodiments of hinge elements.

### Detailed description of the invention

The present invention concerns a device and a method for transmitting torque. The invention has been summarized in the corresponding section. In what follows, the invention is described in detail, preferred embodiments are discussed, and the invention is illustrated by means of examples.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

In a first aspect, the invention provides a device. The device comprises a transmission system comprising an elongated and bendable (lengthwise flexible) torque transmission member and a hinge element along which the member is guided, preferably passively guided, via one or more guide pulleys. The hinge element comprises a first and a second base element hingedly attached and comprising a first hinge axis. The first base element comprises a first guide pulley comprising a pulley rotational axis in essence perpendicular to the first hinge axis. The second base element comprises a second guide pulley comprising a pulley rotational axis in essence perpendicular to the first hinge axis. The first guide pulley comprises a position within the first base element. The second guide pulley comprises a position within the second base element. The positions of both the first guide pulley within the first base element and the second guide pulley within the second base element are configured to guide a portion of the member in essence coaxial to the first hinge axis.

In a second aspect, the present invention provides a method for transmitting torque, preferably in a device, from an input rotational axis to an output rotational axis which is nonparallel with the input rotational axis via an elongated and bendable (lengthwise flexible) torque transmission member. The method comprises the simultaneous steps of relatively rotating the input and output rotational axes about at least one hinge axis; and guiding for each hinge axis of the at least one hinge axis a portion of the member in essence coaxial to the hinge axis. Preferably, each hinge axis of the at least one hinge axis is spatially separated from both of the input and output rotational axes. "Spatially separated from" hereby means "comprising a nonzero distance with". In this aspect, a device is provided which comprises the input rotational axis, the output rotational axis, the elongated and bendable torque transmission member, and the at least one hinge axis.

In a preferred embodiment, the method further comprises the steps of guiding a portion of the member in essence perpendicular to the input rotational axis from an input pulley comprising the input rotational axis to a hinge axis of the at least one hinge axis; and guiding a portion of the member in essence perpendicular to said output rotational axis from a hinge axis of the at least one hinge axis to an output pulley comprising the output rotational axis. In this preferred embodiment, the device further comprises the input pulley and the output pulley.

The present invention provides a solution to the technical problem of torque transmission over relatively rotatable and nonparallel input and output axes. The present invention is advantageous as it allows for limited spatial extent of a hinge element in a direction perpendicular to the first hinge axis. The present invention is additionally advantageous as the tension on the member, e.g. cable, is in essence independent, up to torsion of the member, of the angular configuration of the hinge element, because the length of the path of the member is in essence independent of the angular configuration of the hinge element.

One of ordinary skill in the art will appreciate that the first and second aspect comprise a common inventive concept. The method according to the second aspect may be carried out via a device according to the first aspect. Therefore, in what follows, reference to a particular aspect may be left out. Furthermore, all features described in this document (above and below) may pertain to each of the first and second aspect, even if they have been described in conjunction with a particular aspect.

A non-limiting list of elongated and bendable torque transmission members comprises a belt, a cable, a chain, and the like. In a preferred embodiment, the member is a cable, preferably a cable comprising an alloy, a metal or a plastic, more preferably a cable comprising a plastic, even more preferably a cable comprising polyethylene (PE), most preferably a cable comprising Dyneema® from DSM Dyneema B.V. Cables are advantageous as they allow for torsion without critically straining the cable, while keeping the length of the path of the member in essence constant.

In a preferred embodiment, the device is a wearable robot. Preferably, the wearable robot is an exoskeleton. In a preferred embodiment, the wearable robot comprises a joint comprising a joint rotational axis and the hinge element. The first hinge axis may or may not be the joint rotational axis. A non-limiting list of joints comprises an ankle, an elbow, a hip, a knee, a shoulder, a wrist, and the like.

One of ordinary skill in the art will appreciate that ordinal numbers, such as "first", "second", "third", "fourth", "fifth", "sixth", "seventh" and "eight", as used in this document refer to elements of the device or steps of the method without implying a total number of such elements or such steps. For example: a hinge element may comprise a first guide pulley, a second guide pulley, a fifth guide pulley and a sixth guide pulley, and may thereby comprise four guide pulleys in total. However, a hinge element comprising a first guide pulley, a second guide pulley, a fifth guide pulley and a sixth guide pulley may alternatively comprise additional guide pulleys, such as a third guide pulley and a fourth guide pulley, for example.

In a preferred embodiment, the member is guided once along the hinge element.

In an alternative preferred embodiment, the member is guided twice along the hinge element over two separate portions. The first base element may comprise a third guide pulley comprising a pulley rotational axis in essence perpendicular to the first hinge axis. The second base element may comprise a fourth guide pulley comprising a pulley rotational axis in essence perpendicular to the first hinge axis. The third guide pulley may comprise a position within the first base element. The fourth guide pulley may comprise a position within the second base element. The positions of both the third guide pulley within the first base element and the fourth guide pulley within the second base element are configured to guide a second portion of the member in essence coaxial to the first hinge axis.

This is advantageous as it allows for guiding a closed-loop member or a member comprising multiple separate sections. This is additionally or alternatively advantageous as double guidance of the member over the hinge element allows to strain two sections of the member (closed-loop or separate sections) in parallel for the desired torque transmission, thereby reducing the requirements on the member, increasing the longevity of the member and/or allowing larger torques to be transmitted.

In a preferred embodiment, the pulley rotational axes of the first and third guide pulleys are in essence parallel. Preferably, the first and third guide pulleys are in essence coplanar. In a preferred embodiment, the pulley rotational axes of the second and fourth guide pulleys are in essence parallel. Preferably, the second and fourth guide pulleys are in essence coplanar. This is advantageous as it allows for a first and a second base element, each of which comprises a limited extent in a direction perpendicular to the first hinge axis. Such a corresponding narrow hinge is advantageous as it limits the spatial extent of the device. If the device is a wearable robot, a limited spatial extent of the device limits the inertial forces felt by a user.

In a preferred embodiment, the device comprises an input pulley and an output pulley mounted on different base elements, whereby the member is engaged over the input and output pulleys. The input pulley comprises an input rotational axis. The output pulley comprises an output rotational axis. The device is configured for torque transmission from the input rotational axis to the output rotational axis via the elongated and bendable torque transmission member, whereby the input and output rotational axes may relatively rotate, preferably about a first hinge axis in essence perpendicular to both of the input and output rotational axes. The member may be attached to the input pulley. The member may be attached to the output pulley. This is advantageous as it prevents slipping. The output and input pulleys may comprise a transmission ratio smaller than 1, equal to 1 or larger than 1. The transmission ratio may be about 1:3, about 1:2, about 2:3, about 1:1, about 3:2, about 2:1, about 3:1, or any value below, in between or above. The transmission ratio may be different from 1, preferably smaller than 19:20 or larger than 20:19, more preferably smaller than 9:10 or larger 10:9, even more preferably smaller than 4:5 or larger than 5:4 This is advantageous as it allows to alter torques and rotational speeds via transmission over the input and output pulleys.

In a preferred embodiment, the first base element comprises the input pulley. Preferably, the input pulley comprises an input rotational axis in essence perpendicular to the first hinge axis. Preferably, the rotational axes of the first guide pulley and the input pulley are in essence parallel. Preferably, the first guide pulley and the input pulley are in essence coplanar.

In a preferred embodiment, the second base element comprises the output pulley. Preferably, the output pulley comprises an output rotational axis in essence perpendicular to the first hinge axis. Preferably, the rotational axes of the second guide pulley and the output pulley are in essence parallel. Preferably, the second guide pulley and the output pulley are in essence coplanar.

In an alternative preferred embodiment, the hinge element comprises multiple hinge axes. The multiple hinge axes may comprise in essence parallel hinge axes. The multiple hinge axes may comprise nonparallel hinge axes. The multiple hinge axes may comprise in essence perpendicular hinge axes.

In a preferred embodiment, the hinge element comprises the first, the second and a third base element, whereby the second and the third base element are hingedly attached and comprise a second hinge axis. The second base element may comprise a fifth guide pulley comprising a pulley rotational axis in essence perpendicular to the second hinge axis. The third base element may comprise a sixth guide pulley comprising a pulley rotational axis in essence perpendicular to the second hinge axis. The fifth guide pulley may comprise a position within the second base element. The sixth guide pulley may comprise a position within the third base element. The positions of both the fifth guide pulley within the second base element and the sixth guide pulley within the third base element are configured to guide a portion of the member in essence coaxial to the second hinge axis. Preferably, the first base element comprises an input pulley comprising an input rotational axis in essence parallel to the pulley rotational axis of the first guide pulley. Preferably, the input pulley and the first guide pulley are in essence coplanar. Preferably, the third base element comprises an output pulley comprising an output rotational axis in essence parallel to the pulley rotational axis of the sixth guide pulley. Preferably, the output pulley and the sixth guide pulley are in essence coplanar. Preferably, the member is engaged over the input and output pulleys.

The first and the second hinge axis may be in essence parallel. The first and the second hinge axis may be nonparallel. The first and the second hinge axis may be in essence perpendicular. The latter two cases are advantageous at it allows to relatively hinge the input and output axes around multiple directions. In a preferred embodiment, the first and the second hinge axis are in essence perpendicular.

In a preferred embodiment, the fifth guide pulley is the second guide pulley. The second guide pulley thereby comprises a pulley rotational axis in essence perpendicular to both of the first and the second hinge axis.

The device may or may not comprise a flexible drive shaft. In a preferred embodiment, the device comprises a flexible drive shaft in series with the input or output pulley of the hinge element. A flexible drive shaft is advantageous as it allows for torque transmission along nonparallel axes, may be utilized as a compliant element in, for example, a series elastic actuator, and allows for decentralized torque transmission from a remote actuation system. A flexible drive shaft and the hinge element are complementary elements in a device: the flexible drive shaft does not by itself provide support, but may be flexed in many directions along multiple portions, while the hinge element can provide support, for example along a direction parallel to the first hinge axis, but comprises a limited amount of degrees of freedom. This allows a flexible drive shaft to be utilized in combination with supportive elements of a wearable robot, or, if intact, with naturally present supportive elements of a human or animal being. Furthermore, while a flexible drive shaft can be utilized as a compliant element, its inertia in transmitting torque may also be disadvantageous in other cases, where immediate reaction is desired, a problem solved by the hinge element of a device according to the present invention.

In an embodiment, one or more pulleys, such as the input pulley, the output pulley and/or a guide pulley may be a wheel, e.g. a geared wheel. Additionally or alternatively the input pulley, output pulley and/or guide pulley may comprise a profile deviating from circular, such as a cam profile, as long as the pulleys are rotationally configured to keep the length of the member constant. This allows to have a variable transmission.

The invention is further described by the following non-limiting examples which further illustrate the invention, and are not intended to, nor should they be interpreted to, limit the scope of the invention.

### Examples

### Example 1: Torque transmission with two degrees of freedom (2 DOF)

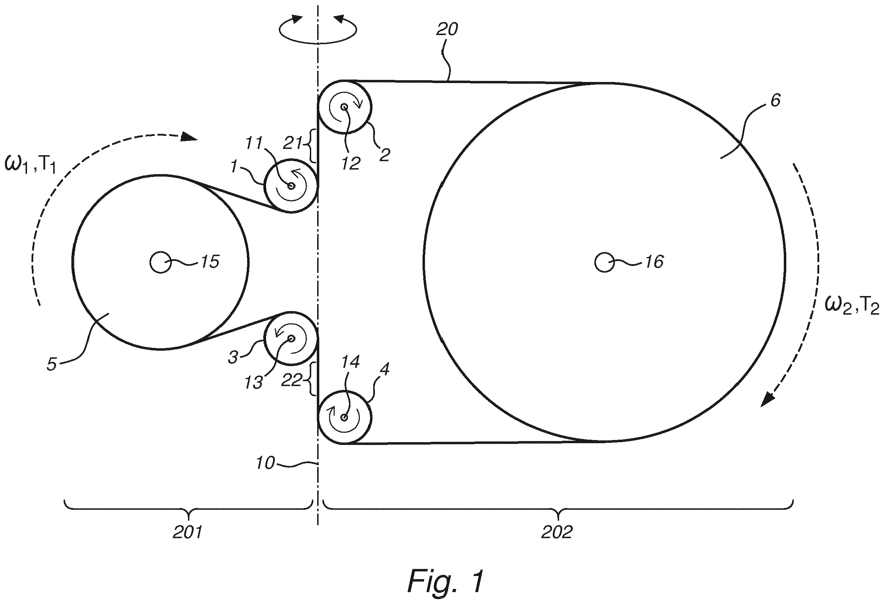

Figure 1 shows a schematic representation of an embodiment of a hinge element of a device according to the present invention. The hinge element comprises a first base element (201) and a second base element (202) which are hingedly attached and which comprise a first hinge axis (10).

The first base element (201) comprises an input pulley (5), a first guide pulley (1), and a third guide pulley (3). The input pulley (5) comprises an input rotational axis (15). The first guide pulley (1) comprises a pulley rotational axis (11). The third guide pulley (3) comprises a pulley rotational axis (13). The input pulley (5), first guide pulley (1) and third guide (3) pulley are in essence coplanar, thereby comprising in essence parallel rotational axes (15, 11, 13) which are in essence perpendicular to the first hinge axis (10).

The second base element (202) comprises an output pulley (6), a second guide pulley (2), and a fourth guide pulley (4). The output pulley (6) comprises an output rotational axis (16). The second guide pulley (2) comprises a pulley rotational axis (12). The fourth guide pulley (4) comprises a pulley rotational axis (14). The output pulley (6), second guide pulley (2) and fourth guide pulley (4) are in essence coplanar, thereby comprising in essence parallel rotational axes (16, 12, 14) which are in essence perpendicular to the first hinge axis (10).

The device, and in particular the hinge element, furthermore comprises an elongated and bendable torque transmission member (20) which is engaged over each of the input pulley (5) and the output pulley (6), and which is furthermore guided, preferably passively guided, along the hinge element via the first (1), second (2), third (3) and fourth (4) guide pulleys. The positions of the first guide pulley (1) within the first base element (201) and the second guide pulley (2) within the second base element (202) are configured to guide a first portion (21) of the member in essence coaxial to the first hinge axis (10). The positions of the third guide pulley (3) within the first base element (201) and the fourth guide pulley (4) within the second base element (202) are configured to guide a second portion (22) of the member in essence coaxial to the first hinge axis (10).

The first pulley (1) comprises a first cable guidance radius and the pulley rotational axis of the first pulley (1) is positioned within the first base element (201) at a distance from the first hinge axis (10) in essence equal to the first cable guidance radius. The third pulley (3) comprises a third cable guidance radius and the pulley rotational axis of the third pulley (3) is positioned within the first base element (201) at a distance from the first hinge axis (10) in essence equal to the third cable guidance radius. Preferably, the first and third cable guidance radii are in essence equal.

The second pulley (2) comprises a second cable guidance radius and the pulley rotational axis of the second pulley (2) is positioned within the second base element (202) at a distance from the first hinge axis (10) in essence equal to the second cable guidance radius. The fourth pulley (4) comprises a fourth cable guidance radius and the pulley rotational axis of the fourth pulley (4) is positioned within the second base element (202) at a distance from the first hinge axis (10) in essence equal to the fourth cable guidance radius. Preferably, the second and fourth cable guidance radii are in essence equal.

The hinge element comprises two degrees of freedom:
- a common rotational degree of freedom (ω1, T1, ω2, T2) of the input (15) and output (16) rotational axes; and
- a passive rotational degree of freedom of the input (15) and output (16) rotational axes about the first hinge axis (10).

One of ordinary skill in the art will appreciate that while arrows are drawn in conjunction with the pulleys in Figure 1, the rotational direction is not limited to the direction as drawn, but merely serves to indicate a common rotational motion caused by the member. All pulleys are configured to rotate bidirectional.

### Example 2: Torque transmission with multiple parallel hinge axes

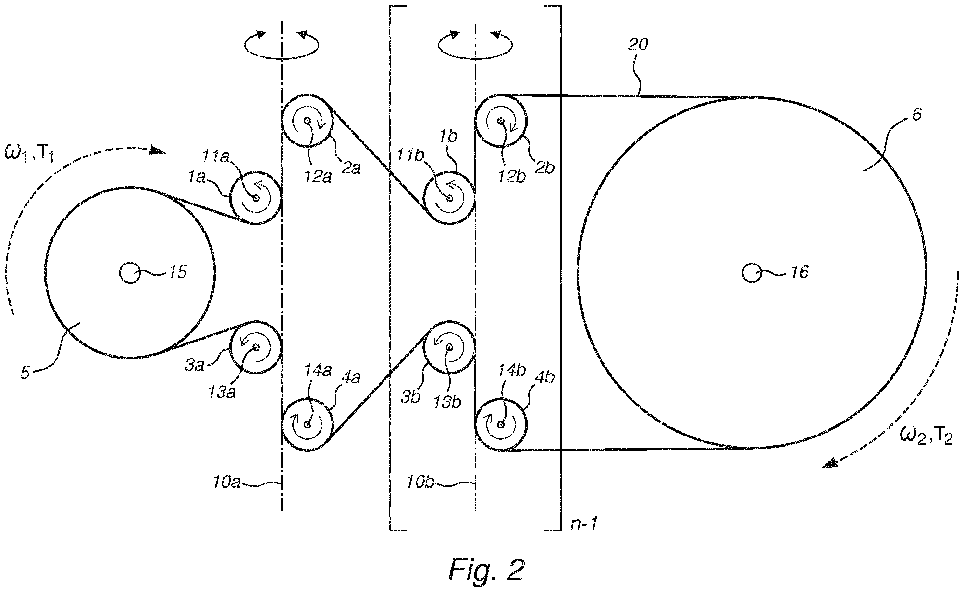

Figure 2 shows a schematic representation of an embodiment of a hinge element of a device according to the present invention. The hinge element comprises multiple in essence parallel hinge axes (10a, 10b), whereby for each hinge axis the hinge element comprises two pairs of guide pulleys:
- pair (1a, 2a) and pair (3a, 4a) for hinge axis (10a); and
- pair (1b, 2b) and pair (3b, 4b) for hinge axis (10b).

The guide pulleys of each pair are positioned to guide a portion of the member in essence coaxial to the corresponding hinge axis.

The feature numbering corresponds to the feature numbering of example 1, whereby one of ordinary skill in the art will appreciate that alphabetic indexing per hinge axis has been utilized. The present example comprises n hinge axes and therefore comprises n+1 DOF. All other features as disclosed in conjunction with example 1, are also present in this example.

### Example 3: Torque transmission with two nonparallel hinge axes

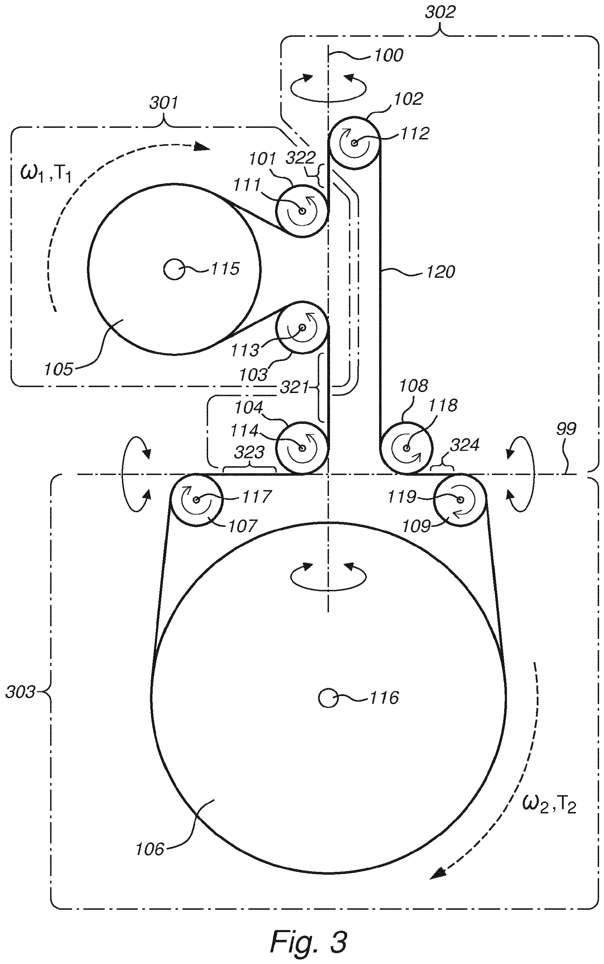

Figure 3 shows a schematic representation of an embodiment of a hinge element of a device according to the present invention. The hinge element comprises a first base element (301), a second base element (302), and a third base element (303). The first base element (301) and the second base element (302) are hingedly attached and comprise a first hinge axis (100). The second base element (302) and the third base element (303) are hingedly attached and comprise a second hinge axis (99).

The first base element (301) comprises an input pulley (105), a first guide pulley (103), and a third guide pulley (101). The input pulley (105) comprises an input rotational axis (115). The first guide pulley (103) comprises a pulley rotational axis (113). The third guide pulley (101) comprises a pulley rotational axis (111). The input pulley (105), first guide pulley (103) and third guide pulley (101) are in essence coplanar, thereby comprising in essence parallel rotational axes (115, 113, 111) which are in essence perpendicular to the first hinge axis (100).

The second base element (302) comprises a second guide pulley (104), a fourth guide pulley (102) and a seventh guide pulley (108). The second guide pulley (104) comprises a pulley rotational axis (114). The fourth guide pulley (102) comprises a pulley rotational axis (112). The seventh guide pulley (108) comprises a pulley rotational axis (118). The second (104), fourth (102) and seventh (108) guide pulleys are in essence coplanar, thereby comprising in essence parallel rotational axes (114, 112, 118) which are in essence perpendicular to each of the first (100) and the second (99) hinge axis.

The third base element (303) comprises an output pulley (106), a sixth guide pulley (107) and an eighth guide pulley (109). The output pulley (106) comprises an output rotational axis (116). The sixth guide pulley (107) comprises a pulley rotational axis (117). The eighth guide pulley (109) comprises a pulley rotational axis (119). The output pulley (106), sixth guide pulley (107) and eighth guide pulley (109) are in essence coplanar, thereby comprising in essence parallel rotational axes (116, 117, 119) which are in essence perpendicular to the second hinge axis (99).

The device, and in particular the hinge element, furthermore comprises an elongated and bendable torque transmission member (120) which is engaged over each of the input pulley (105) and the output pulley (106), and which is furthermore guided, preferably passively guided, along the hinge element via the first (104), second (104), third (101), fourth (102), sixth (107), seventh (108) and eighth (109) guide pulleys. One of ordinary skill in the art will appreciate that the embodiment comprises seven guide pulleys, and that according to a preferred embodiment described in the detailed description, the fifth guide pulley and the second guide pulley (104) are one and the same.

The hinge element comprises several pairs of guide pulleys:
- the third (101) and fourth (102) guide pulleys form a pair guiding portion (321) of the member in essence coaxial to the first hinge axis (100);
- the first (103) and second (104) guide pulleys form a pair guiding portion (322) of the member in essence coaxial to the first hinge axis (100);
- the second (104) and sixth (107) guide pulleys form a pair guiding portion (323) of the member in essence coaxial to the second hinge axis (99); and
- the seventh (108) and eighth (109) guide pulleys form a pair guiding portion (324) of the member in essence coaxial to the second hinge axis (99).

The positions of each guide pulley of a pair within its corresponding first, second or third base element of the hinge element is configured to guide a portion of the member in between the guide pulleys of the pair in essence coaxial to the corresponding first or second hinge axis. Each guide pulley may thereby comprise a cable guidance radius, whereby the guide pulley is positioned within its corresponding first, second or third base element at a distance from the corresponding first or second hinge axis in essence equal to the cable guidance radius.

The hinge element comprises three degrees of freedom (3 DOF):
- a common rotational degree of freedom (ω1, T1, ω2, T2) of the input (15) and output (16) rotational axes;
- a passive rotational degree of freedom of the first (301) and the second (302) base element about the first hinge axis (100); and
- a passive rotational degree of freedom of the second (302) and the third (303) base element about the second hinge axis (99).

The input (15) and output (16) rotational axes may hence relatively rotate about two nonparallel hinge axes. Preferably, the first (100) and the second (99) hinge axes are in essence perpendicular.

One of ordinary skill in the art will appreciate that while arrows are drawn in conjunction with the pulleys in Figure 3, the rotational direction is not limited to the direction as drawn, but merely serves to indicate a common rotational motion caused by the member. All pulleys are configured to rotate bidirectional.

### Example 4: Embodiment corresponding to example 1

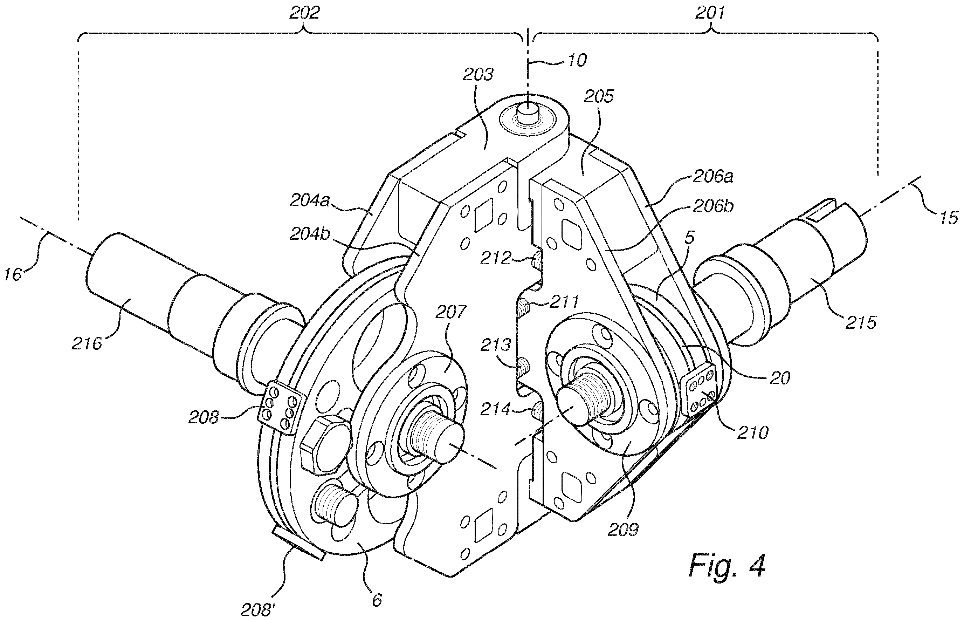

Figure 4 shows a perspective view of an embodiment of a hinge element of a device according to the present invention, which corresponds to the first example, and which therefore comprises 2 DOF. One of ordinary skill in the art will appreciate that likewise numbered features in examples 1 and 4 correspond. The hinge element comprises the first base element (201) and the second base element (202) which are hingedly attached and comprise a first hinge axis (10).

The first base element (201) comprises two in essence parallel plates (206a, 206b), whereby in between the two plates (206a, 206b) the input pulley (5), first guide pulley and third guide pulley are rotatably attached. Each of these pulleys (5, 1, 3) comprises a rotational axis perpendicular to the first hinge axis (10) and perpendicular to each of the two plates (206a, 206b). The first base element (201) furthermore comprises an input shaft (215) coaxial with the input rotational axis (15) of the input pulley (5) and connected to the input pulley (5). Each of the plates (206a, 206b) comprises a holder plate (209) comprising bearings for the input shaft (215). The connector shafts (211, 213) of the first and third guide pulley are attached to each of the two plates (206a, 206b) and are configured for rotation of the guide pulleys about the connector shafts.

The second base element (202) comprises two in essence parallel plates (204a, 204b), where in between the two plates (204a, 204b) the output pulley (6), second guide pulley and fourth guide pulley are rotatably attached. Each of these pulleys (6, 2, 4) comprises a rotational axis perpendicular to the first hinge axis (10) and perpendicular to each of the two plates (204a, 204b). The second base element (202) furthermore comprises an output shaft (216) coaxial with the output rotational axis (16) of the output pulley (6) and connected to the output pulley (6). Each of the plates (204a, 204b) comprises a holder plate (207) comprising bearings for the output shaft (216). The connector shafts (212, 214) of the second and fourth guide pulley are attached to each of the two plates (204a, 204b) and are configured for rotation of the guide pulleys about the connector shafts.

The hinge element furthermore comprises a cable (20) which is attached to each of the input and output pulleys (5, 6) via connector plates (208, 208', 210). The system may furthermore comprise a tensioning (pre-compression) mechanism for the cable (20). Preferably the output pulley (6) comprises the tensioning mechanism. In a particular embodiment of the tensioning mechanism, the tensioning mechanism exerts a force on one or both of the two connector plates (208, 208') for tensioning the cable (20). The input and output pulleys thereby comprise a limited range of rotational motion. However, for certain joints, e.g. a knee, a limited range of rotational motion (e.g. 150°) does not pose a restriction in practice.

The first base element (201) comprises a pair of connector blocks (205) and the second base element (202) comprises a pair of connector blocks (203). The connector blocks (205) of the first base element (201) are mounted in between the two plates (206a, 206b) of the first base element (201). The connector blocks (203) of the second base element (202) are mounted in between the two plates (204a, 204b) of the second base element (202). A connector block (205) of the first base element (201) comprises a cylindrical protrusion. A connector block (203) of the second base element (202) comprises a cylindrical cavity. The cylindrical protrusion is rotatably engaged in the cylindrical cavity in order to hingedly attach the first (201) and the second (202) base element. The cylindrical protrusion and the cylindrical cavity thereby comprise a common rotational axis, which is equal to the first hinge axis (10).

### Example 5: Wearable robot

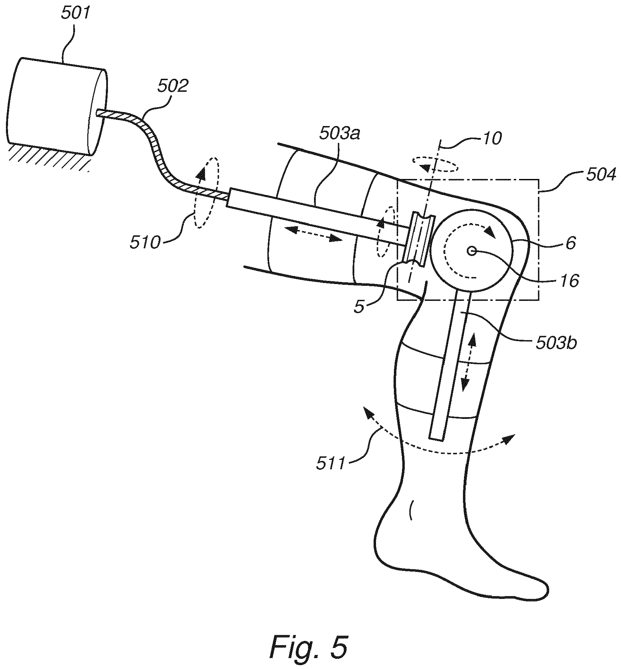

Figure 5 shows a schematic representation of a portion of a wearable robot according to the present invention. The wearable robot comprises, in series, a remote actuation system (501) such as a motor, a flexible drive shaft (502), a telescopically configured rigid thigh shaft (503a) extending along a thigh, a hinge element according to example 1 or example 4, and a telescopically configured rigid lower leg shaft (503b) extending along a lower leg. The input pulley thereby comprises an input rotational axis extending along the thigh. The rigid thigh shaft (503a) is connected in essence parallel to the input rotational axis to the input pulley. The first hinge axis is preferably in essence parallel to a plane defined by the thigh and the lower leg. The output pulley drives knee rotational motion, i.e. relative motion of the lower leg shaft with respect to the thigh. The rigid lower leg shaft is thereby connected to the output pulley perpendicularly to the output rotational axis.

Torque exerted by the remote actuation system (501) results in rotation (510) of the flexible drive shaft (502) and the rigid thigh shaft (503a), and, via the hinge element, in rotation (511) of the rigid lower leg shaft (503b) with respect to the rigid thigh shaft (503a). The passive degrees of freedom of the transmission system (the hinges) are also beneficial as they provide a means to compensate misalignments between the center of rotation of the human joints and the output pulley in this case, improving the torque transference from the robot to the human and the safety of the device.

A flexible drive shaft is advantageous as it allows for torque transmission along nonparallel axes, may be utilized as a compliant element in, for example, a series elastic actuator, and allows for decentralized torque transmission. In the present example, the remote actuation system (501) may be attached to a rump and the flexible drive shaft (502) enables torque transmission over the hip to the rigid thigh shaft (503a).

### Example 6: Single guidance and torsion spring

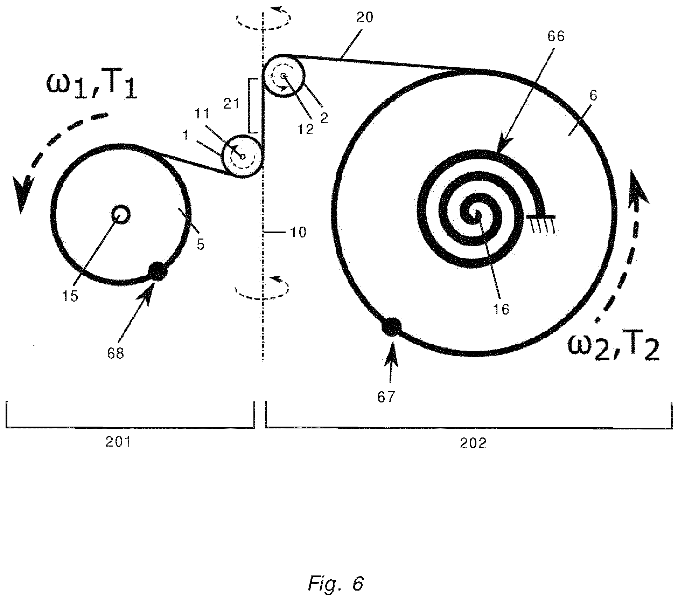

Figure 6 shows a schematic representation of an embodiment of a hinge element of a device according to the present invention. The hinge element comprises two degrees of freedom:
- a common rotational degree of freedom (ω1, T1, ω2, T2) of the input (15) and output (16) rotational axes; and
- a passive rotational degree of freedom of the input (15) and output (16) rotational axes about the first hinge axis (10).

The feature numbering in Figure 6 corresponds to the feature numbering in Figure 1 and the description in example 1. Whereas the member is closed-loop in example 1 and thereby guided twice along different portions in essence coaxial to the hinge axis, the member (20) is guided only once from input to output pulley. The member is attached at a first attachment portion (68) to the input pulley (5) and at a second attachment portion (67) to the output pulley (6). The device furthermore comprises a torsion spring (66) for returning to an initial rotational position. The device is suitable for unidirectional torque transmission.

One of ordinary skill will appreciate that while the torsion spring (66) is connected to the output pulley in Figure 6, additionally or alternatively a torsion spring (66) may be connected to the input pulley.

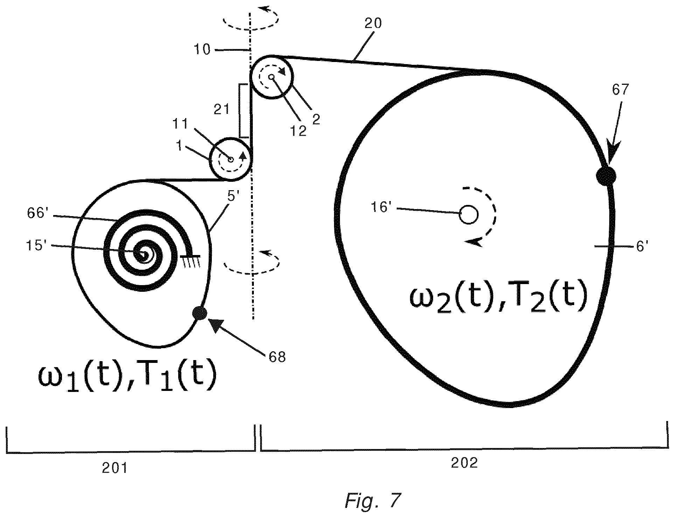

Figure 7 shows a schematic representation of an embodiment of a hinge element of a device according to the present invention. The hinge element comprises two degrees of freedom:
- a common rotational degree of freedom (ω1, T1, ω2, T2) of the input (15') and output (16') rotational axes; and
- a passive rotational degree of freedom of the input (15') and output (16') rotational axes about the first hinge axis (10).

The feature numbering in Figure 7 corresponds to the feature numbering in Figure 6 and the description above. The device comprises a torsion spring (66) for returning to an initial rotational position, which is connected to the input pulley (5') instead of the output pulley (6'). In addition, the input and output pulleys comprise a cam profile, and are rotationally configured to keep the length of the member (20) constant. This allows for a variable transmission.

### Example 7: Embodiment with variable output stiffness

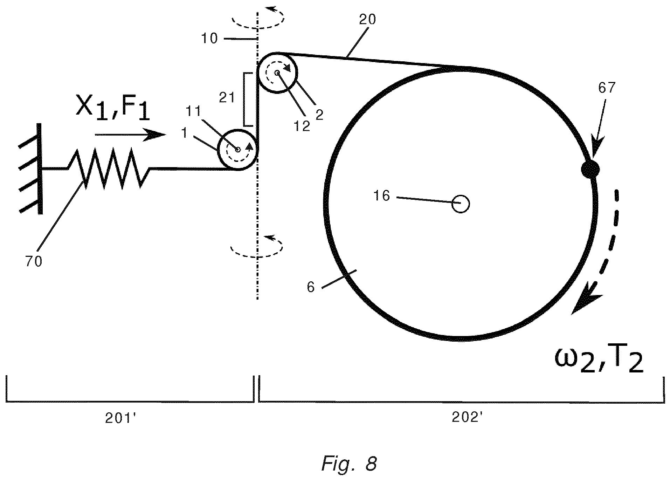

Figures 8 and figure 9 show schematic representations of a device comprising an elongated and bendable torque transmission member (20) and a hinge element along which the member is guided via one or more guide pulleys (1, 2). The hinge element comprises a first (201') and a second (202') base element hingedly attached and comprising a first hinge axis (10). The first base element comprises a first guide pulley (1) with pulley rotational axis (11) in essence perpendicular to the first hinge axis. The second base element comprises a second guide pulley (2) with pulley rotational axis (12) in essence perpendicular to the first hinge axis. The first guide pulley (1) comprises a circular profile and a first member guidance radius, whereby the pulley rotational axis (11) of the first guide pulley (1) is positioned within the first base element (201') at a distance from the first hinge axis (10) in essence equal to the first member guidance radius. The second guide pulley (2) comprises a circular profile and a second member guidance radius, whereby the pulley rotational axis (12) of the second guide pulley (2) is positioned within the second base element (202') at a distance from the first hinge axis (10) in essence equal to the second member guidance radius. The positioning of the first guide pulley (1) within the first base element (201') and the second guide pulley (2) within the second base element (202') are thereby configured to guide a portion (21) of the member (20) in essence coaxial to the first hinge axis (10). The first base element (201') comprises a spring (70) to which the member (20) is attached. The second base element (202') comprises an output pulley (6, 6') to which the member (20) is attached at a second attachment portion (67). The output pulley comprises an output rotational axis (16, 16'). The output pulley may comprise a circular profile (output pulley (6) in Figure 8) or a noncircular profile (output pulley (6') in Figure 9).

In a preferred embodiment in case of a noncircular profile, the noncircular profile is a cam profile. In a preferred embodiment, the device is a wearable robot. Preferably, the wearable robot is an exoskeleton.

The present embodiment is advantageous as the spring (70) allows to create a variable stiffness in the output rotational axis. In addition, the spring ensures that the member is always tensioned.

## Claims

1. Wearable robot comprising an elongated and bendable torque transmission member (20) and a hinge element along which the member is guided via one or more guide pulleys, the hinge element comprising a first (201) and second (202) base element hingedly attached and comprising a first hinge axis (10), **characterized in that,** the first base element comprises a first guide pulley (1) with pulley rotational axis (11) in essence perpendicular to the first hinge axis, the second base element comprises a second guide pulley (2) with pulley rotational axis (12) in essence perpendicular to the first hinge axis, whereby the positions of the first guide pulley within the first base element and the second guide pulley within the second base element are configured to guide a portion (21) of the member in essence coaxial to the first hinge axis.

2. Wearable robot according to preceding claim 1, wherein the member is guided twice along the hinge element over two separate portions, wherein the first base element comprises a third guide pulley (3) with pulley rotational axis (13) in essence perpendicular to the first hinge axis, wherein the second base element comprises a fourth guide pulley (4) with pulley rotational axis (14) in essence perpendicular to the first hinge axis, whereby the positions of the third guide pulley within the first base element and the fourth guide pulley within the second base element are configured to guide a second portion (22) of the member in essence coaxial to the first hinge axis.

3. Wearable robot according to preceding claim 2, wherein the first and third guide pulleys are in essence coplanar and the pulley rotational axes of the first and third guide pulleys are in essence parallel, and wherein the second and fourth guide pulleys are in essence coplanar and the pulley rotational axes of the second and fourth guide pulleys are in essence parallel.

4. Wearable robot according to any one of the preceding claims, wherein the wearable robot comprises an input pulley (5) and an output pulley (6) mounted on different base elements, whereby the member is engaged over the input and output pulleys.

5. Wearable robot according to preceding claim 4, wherein the first base element comprises the input pulley and the input pulley comprises an input rotational axis (15) in essence perpendicular to the first hinge axis.

6. Wearable robot according to preceding claim 5, wherein the first guide pulley and the input pulley are in essence coplanar and the rotational axes of the first guide pulley and the input pulley are in essence parallel.

7. Wearable robot according to any one of preceding claims 4 to 6, wherein the second base element comprises the output pulley and the output pulley comprises an output rotational axis (16) in essence perpendicular to the first hinge axis.

8. Wearable robot according to preceding claim 7, wherein the second guide pulley and the output pulley are in essence coplanar and the rotational axes of the second guide pulley and the output pulley are in essence parallel.

9. Wearable robot according to any one of the preceding claims 4 to 8, wherein the member is attached to the input pulley and/or to the output pulley.

10. Wearable robot according to any one of the preceding claims 4 to 9, wherein the output and input pulleys comprise a transmission ratio equal to or different from, preferably different from, 1:1.

11. Wearable robot according to preceding claim 1, comprising the elongated and bendable torque transmission member (120) and the hinge element along which the member is guided via one or more guide pulleys, the hinge element comprising the first (301), the second (302) and a third (303) base element, whereby:
- the first and the second base element are hingedly attached and comprise a first hinge axis (100);
- the second and the third base element are hingedly attached and comprise a second hinge axis (99);
- the first base element comprises the first guide pulley (103) with pulley rotational axis (113) in essence perpendicular to the first hinge axis, the second base element comprises the second guide pulley (104) with pulley rotational axis (114) in essence perpendicular to the first hinge axis, whereby the positions of the first guide pulley within the first base element and the second guide pulley within the second base element are configured to guide a portion (321) of the member in essence coaxial to the first hinge axis; and
- the second base element comprises a fifth guide pulley (104, 108) with pulley rotational axis (114, 118) in essence perpendicular to the second hinge axis, the third base element comprises a sixth guide pulley (107, 109) with pulley rotational axis (117, 119) in essence perpendicular to the second hinge axis, whereby the positions of the fifth guide pulley within the second base element and the sixth guide pulley within the third base element are configured to guide a portion (323, 324) of the member in essence coaxial to the second hinge axis,
whereby preferably the first hinge axis and the second hinge axis are in essence perpendicular.

12. Wearable robot according to preceding claim 11, wherein the fifth guide pulley is the second guide pulley (104), and whereby the second guide pulley (104) comprises a pulley rotational axis (114) in essence perpendicular to both of the first and the second hinge axis.

13. Device comprising an elongated and bendable torque transmission member (20) and a hinge element along which the member is guided via one or more guide pulleys, the hinge element comprising a first (201) and second (202) base element hingedly attached and comprising a first hinge axis (10), **characterized in that, the** first base element comprises a first guide pulley (1) with pulley rotational axis (11) in essence perpendicular to the first hinge axis, the second base element comprises a second guide pulley (2) with pulley rotational axis (12) in essence perpendicular to the first hinge axis, whereby the positions of the first guide pulley within the first base element and the second guide pulley within the second base element are configured to guide a portion (21) of the member in essence coaxial to the first hinge axis.

14. Method for transmitting torque, preferably in a wearable robot, from an input rotational axis (15, 115) to a parallel or nonparallel, preferably nonparallel, output rotational axis (16, 116) via an elongated and bendable torque transmission member (20, 120), comprising the simultaneous steps of:
- relatively rotating the input and output rotational axes about at least one hinge axis (10, 10a, 10b, 100, 99); and
- guiding for each hinge axis of the at least one hinge axis a portion of the member in essence coaxial to the hinge axis.

15. Method according to preceding claim 14, comprising the steps of:
- guiding a portion of the member in essence perpendicular to said input rotational axis from an input pulley comprising the input rotational axis to a hinge axis of the at least one hinge axis; and
- guiding a portion of the member in essence perpendicular to said output rotational axis from a hinge axis of the at least one hinge axis to an output pulley comprising the output rotational axis.
